(19) Europäisches Patentamt
European Patent Office
Office européen des brevets

(11) EP 0 773 209 B1

(12) # EUROPÄISCHE PATENTSCHRIFT

(45) Veröffentlichungstag und Bekanntmachung des Hinweises auf die Patenterteilung:
**26.01.2000 Patentblatt 2000/04**

(51) Int Cl.[7]: **C07C 69/587**, C07C 67/00, A61K 7/46, A23L 1/226

(21) Anmeldenummer: **96117602.1**

(22) Anmeldetag: **04.11.1996**

(54) **Ungesättigter Ester**
Unsaturated esters
Esters insaturés

(84) Benannte Vertragsstaaten:
**CH DE ES FR GB IT LI NL**

(30) Priorität: **10.11.1995 CH 319195**

(43) Veröffentlichungstag der Anmeldung:
**14.05.1997 Patentblatt 1997/20**

(73) Patentinhaber: **GIVAUDAN-ROURE (INTERNATIONAL) S.A.**
**1214 Vernier, Genève (CH)**

(72) Erfinder: **Kaiser, Roman**
**8610 Uster (CH)**

(74) Vertreter: **Buntz, Gerhard et al**
**GIVAUDAN-ROURE (INTERNATIONAL) SA,**
**Postfach 3255**
**4002 Basel (CH)**

(56) Entgegenhaltungen:
**EP-A- 0 009 632**

- **JOURNAL OF ORGANIC CHEMISTRY, Bd. 55, Nr. 11, 25.Mai 1990, EASTON US, Seiten 3440-3442, XP002023930 MITSUO NAGAI ET AL.: "Syntheses of Bicyclo(3.3.0)octanes via Bifurcating Radical Cyclization Pathways"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 773 209 B1

## Beschreibung

Die Erfindung betrifft die Verbindung Methyl (E)-4,7-octadienoat [(E)-4,7-Octadiensäuremethylester] sowie die Verbindung als organoleptischen Wirkstoff enthaltende Riech- und/oder Geschmackstoffkompositionen und die Herstellung der Verbindung.

Einige Verbindungen, die der erfinderischen Verbindung strukturell nahestehen sind bereits bekannt. So beschrieben M. Nagai et al., 1990, J. Org. Chem., 55, 3440-3442, Ethylester der Formel

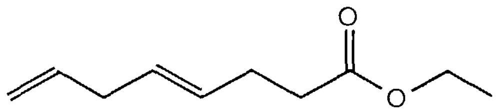

4

Auf irgendwelche olfaktorischen Eigenschaften wird jedoch nicht hingewiesen.

Ferner wurde die Verbindung Ethyl (Z)-4,7-octadienoat der Formel

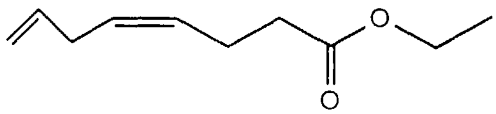

5

von M. Winter et al., 1979, Helv. Chim. Acta, 19, 135-139, im Verlauf gaschromatographischer Isolierung von Aromastoffen der roten Passionsfrucht (Passiflora edulis SIMS) als sensorisch aktive Spurenkomponente von auffallend fruchtigem Geruch mit frischer, saftiger, für Ananas typischer Kopfnote erkannt und synthetisiert. Die Verbindung 5 ist zudem schwer zugänglich, denn ihr Aufbau erfolgt nur über eine anspruchsvolle mehrstufige Synthese. Im Gegensatz dazu kann die Verbindung 1 in einfacher Weise durch eine Orthoesterumlagerung erhalten werden, wie dies weiter unten dargelegt ist.

Der entsprechende Methylester mit der Formel

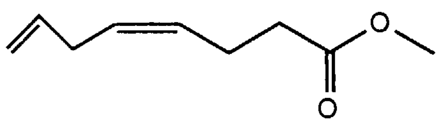

6

ist in der Literatur noch nicht beschrieben worden

Aufgabe der vorliegenden Erfindung ist es, einen neuen organoleptischen Wirkstoff zu finden. Eine zusätzliche Aufgabe besteht darin, insbesondere einen Wirkstoff zu finden, der einen starken, frischen Duft mit frischen, grünaldehydigen Aspekten aufweist, und mit diesem Wirkstoff Riech- und/oder Geschmackstoffkompositionen, insbesondere blumiger, blumig-fruchtiger, frisch-blumiger oder grün-fruchtiger Richtung, zu erstellen.

Die gestellten Aufgaben werden durch Methyl (E)-4,7-octadienoat gelöst.

Die Verbindung Methyl (E)-4,7-octadienoat ist neu und hat die Formel

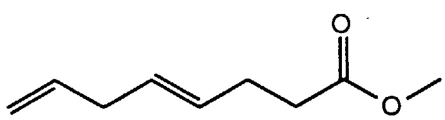

1

Methyl (E)-4,7-octadienoat (1) weist einen sehr starken und frischen Duft auf, der durch grüne und aldehydige Aspekte dominiert wird.

Aufgrund dieser wertvollen olfaktorischen Eigenschaften eignet sich die Verbindung **1** als Riech- und/oder Geschmacksstoff, und zwar insbesondere in Kombination mit der heutzutage zur Verfügung stehenden umfangreichen Palette von natürlichen und synthetischen Riechstoffen bzw. Geschmacksstoffen zur Kreation von Parfüm- bzw. Aroma-Kompositionen, welche ihre Anwendung in allen üblichen Applikationssegmenten finden können. Beispiele der zahlreichen bekannten Riechstoffingredientien natürlichen oder synthetischen Ursprungs, wobei die Palette der natürlichen Rohstoffe sowohl leicht- als auch mittel- und schwerflüchtige Komponenten, und diejenige der Synthetika Vertreter aus etlichen Stoffklassen umfassen kann, sind:

- Naturprodukte, wie Baummoos-Absolue, Basilikumöl, Agrumenöle (wie Bergamotteöl, Mandarinenöl, usw.), Mastix-Absolue, Myrtenöl, Palmarosaöl, Patchouliöl, Petitgrainöl, Wermutöl, Lavendelöl, Rosenöl, Jasminöl, Ylang-Ylangöl, Sandelholzöl,

- Alkohole, wie Farnesol, Geraniol, Linalool, Nerol, Phenyläthylalkohol, Rhodinol, Zimtalkohol, cis-3-Hexenol, Menthol, α-Terpineol,

- Aldehyde, wie Citral, α-Hexylzimtaldehyd, Hydroxycitronellal, Lilial® (p-tert.-Butyl-α-methyl-dihydrozimtaledehyd), Methylnonylacetaldehyd, Phenylacetaldehyd, Anisaldehyd, Vanillin,

- Ketone, wie Allyljonon, α-Jonon, β-Jonon, Isoraldein (Isomethyl-α-jonon), Verbenone, Nootkaton, Geranylaceton,

- Ester, wie Allylphenoxyacetat, Benzylsalicylat, Cinnamylpropionat, Citronellylacetat, Decylacetat, Dimethylbenzylcarbinylacetat, Aethylacetoacetat, Aethylacetylacetat, cis-3-Hexenylisobutyrat, Linalylacetat, Methyldihydrojasmonat, Styrallylacetat, Vetiverylacetat, Benzylacetat, cis-3-Hexenylsalicylat, Geranylacetat, usw.,

- Lactone, wie γ-Undecalacton, δ-Decalacton, Pentadecan-15-olid,

- verschiedene in der Parfümerie benützte Komponenten, wie Indol, p-Menthan-8-thiol-3-on, Methyleugenol, Eugenol, Anethol.

Die unter Verwendung der Verbindung **1** hergestellten Riechstoffkompositionen, insbesondere solcher blumiger oder blumig-furchtiger Richtung, bestechen besonders durch ihre Originalität.

Aufgrund dieser olfaktorischen Eigenschaften eignet sich die Verbindung **1** insbesondere zur Mitverwendung bei der Kreation der verschiedenartigsten Parfüm-Typen. Erfindungsgemäss stellen blumige und blumigfruchtige Kompositionen ein besonders wichtiges Anwendungsgebiet dar, welche durch Zusatz der Verbindung **1** eine angenehme, sehr natürlich wirkende Frische erhalten, und die Kompositionen werden verstärkt durch fruchtige und zusätzlich durch grüne Aspekte bereichert. Aber auch in anderen Parfümtypen wie solchen mit orientalischem, holzigem oder hesperidenartigem Grundcharakter kann die Verbindung **1** vorteilhaft angewendet werden, wodurch die Kopfnote eine wertvolle Bereicherung erfährt.

Die erfinderische Verbindung **1** ist auch zur Mitverwendung bei der Kreation von Aromen, insbesondere generell von Fruchtaromen, geeignet. So werden z.B. in Aromen vom Typ Apfel, Pfirsich, Papaya, Guava, Kiwi, Mango und Banane und insbesondere vom Typ Birne die grünen und frischen Aspekte sehr positiv und in natürlicher Weise unterstrichen.

Derartige Aromen können beispielsweise zur Erzeugung bzw. Verbesserung, Verstärkung, Steigerung oder Modifizierung von Fruchtaromen, z.B. Mango, Pfirsich, Kokos verwendet werden. Als Anwendungsgebiete dieser Aromen kommen beispielsweise Nahrungsmittel, Genussmittel und Getränke (Nahrungsmittelfertigprodukte) in Frage.

Die ausgeprägten Qualitäten der Verbindung **1** ermöglichen die Verwendung als Aromastoff in geringen Konzentrationen. Eine geeignete Dosierung umfasst den Bereich von 0,01 bis 100 ppm, vorzugsweise von 0,1 bis 10 ppm, im Fertigprodukt, d.h. dem aromatisierten Nahrungsmittel, Genussmittel oder Getränk.

Die Verbindung **1** kann auf übliche Weise mit den für Aromen verwendeten Bestandteilen vermischt bzw. solchen Aromen zugesetzt werden. Unter den erfindungsgemäss verwendeten Aromen werden Geschmackstoffkompositionen verstanden, die sich auf an sich bekannte Art verdünnen bzw. - insbesondere in essbaren Materialien - verteilen lassen. Sie enthalten beispielsweise etwa 0,01 - 30 Gew.%, insbesondere 0,1 - 10 Gew.%, der Verbindung **1**. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmässigerweise verwendeten bekannten Aromastoffe sind entweder in der obigen Zusammenstellung bereits enthalten oder können der Literatur entnommen werden, wie z.B. J. Merory, Food Flavorings, Composition, Manufacture and Use, Second Edition, The Avi Publishing Company, Inc., Westport, Conn. 1968, oder G. Fenaroli, Fenaroli's Handbook of Flavor Ingredients, Second Edition, Volume 2, CRC-Press, Inc. Cleveland, Ohio, 1975.

Für die Herstellung solcher üblicher Gebrauchsformen kommen beispielsweise folgende Trägermaterialien, Verdickungsmittel, Geschmackstoffverbesserer, Gewürze und Hilfsingredientien, usw. in Frage:

Gummi arabicum, Tragant, Salze oder Brauereihefe, Alginate, Carrageen oder ähnliche Absorbentien; Maltol, Gewürzoleoresine, Raucharomen; Gewürznelken, Diacetyl, Natriumcitrat; Mononatriumglutamat, dinatriuminosin-5'-monophosphat (IMP), dinatriumguanosin-5-phosphat (GMP); oder spezielle Aromastoffe, Wasser, Aethanol, Propylenglykol, Glycerin, usw.

Dank ihrer hochstehenden olfaktorischen Eigenschaften eignet sich die Verbindung 1 bevorzugt für die Luxusparfümerie und für Kosmetikanwendungen.

Methyl (E)-4,7-octadienoat (1) ist erfindungsgemäss herstellbar aus 1,5-Hexadien-3-ol (2) durch Orthoester-Claisen-Umlagerung mit Trimethylorthoacetat (3) in Anwesenheit von einer Carbonsäure C1 bis C8, insbesondere in Anwesenheit von Propionsäure, gemäss der Gleichung:

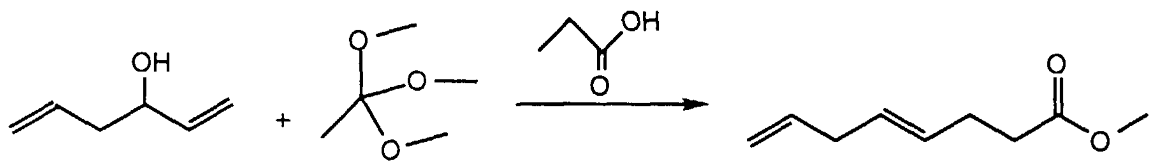

2 3 1

Das dabei benötigte 1,5-Hexadien-3-ol (2) ist bekannt und durch Grignard-reaktion von Allylmagnesiumbromid und Acrolein unter Anwendung von Standardmethoden erhältlich.

Im Rahmen der Syntheseversuche zur Hervorbringung der Erfindung wurden auch einige Derivate von Methyl (E)-4,7-octadienoat 1 synthetisiert, die olfaktorische Eigenschaften aufweisen, nämlich die in der nachfolgenden Tabelle I aufgeführten Methyl(alkyl)-homologen Verbindungen der erfinderischen Verbindung (1).

Tabelle I

| | Verbindung | Geruch |
|---|---|---|
| a) | Methyl (E)-3-methyl-4,7-octadienoat | grün, fettig, leicht fruchtig |
| b) | Methyl (E)-4-methyl-4,7-octadienoat | fruchtig, fettig, pilzig |
| c) | Methyl 3-ethyl-4-methyl-4,7-octadienoat | erdig, pilzig, grün |
| d) | Methyl (E)-3-propyl-4,7-octadienoat | eigenartige, schweissige Fruchtnote, überreife Birne, nicht sehr angenehm |
| e) | Methyl (E)-5-methyl-4,7-octadienoat | grün, fruchtig, fettig |

Ueberraschenderweise wurde festgestellt, dass keine der genannten Verbindungen a) bis e) die hervorragenden Eigenschaften der erfinderischen Verbindung 1, nämlich den frischen Duft mit frischen, grünaldehydigen Aspekten, aufweist. Bei diesen Verbindungen a) bis e) stören vor allem die Geruchsaspekte fettig, pilzig und schweissig.

Weitere Vorteile, Merkmale und Einzelheiten der Erfindung ergeben sich aus den nachfolgenden Beispielen.

Die mit * versehenen Trivialnamen einzelner Komponenten sind bezüglich ihrer systematischen chemischen Bezeichnungen in Standardwerken aufgeführt: z.B. Allured's Flavor and Fragrance Materials - 1996, Allured Publishing Corporation, Carol Stream, Illinois, U.S.A. oder S. Arctander, Perfume and Flavor Chemicals - 1969, published by the author, Montclair, New Jersey, U.S.A.

I. Herstellung von Methyl (E)-4,7-octadienoat

Beispiel 1

78,0 g (ca. 0,71 Mol) 1,5-Hexadien-3-ol, welche durch Grignard-Reaktion von Allylmagnesiumbromid und Acrolein erhalten wurden, werden in 198,0 g (1,65 Mol) Trimethylorthoacetat gelöst und anschliessend 3,0 g (0,04 Mol) Propionsäure zugegeben. Das Reaktionsgemisch wird nun auf 120°C erhitzt und das während der Reaktion gebildete Methanol über eine 15 cm Widmerkolonne abdestilliert. Nach 2 Stunden wird zur Vervollständigung der Reaktion noch während 1 Stunde bei 130°C gehalten, das Reaktionsgemisch dann auf Raumtemperatur abgekühlt, mit 500 ml tert. Butylmethyläther verdünnt, die Lösung zweimal mit 100 ml gesättigter Natriumbicarbonat-Lösung gewaschen, mit Natriumsulfat getrocknet und eingeengt. Die Destillation der so erhaltenen 128 g Rohprodukt über eine 20 cm Widmerkolonne ergibt 51,6 g olfaktorisch gutes Methyl (E)-4,7-octadienoat vom Siedepunkt 76°C/17 mbar und einer Reinheit von ca. 98%.

Spektrale Daten des so hergestellten Methyl (E)-4,7-octadienoat:

NMR (200 MHz, $CDCl_3$): 2,38 (m,4H); 2,73 (m,2H); 3,68 (s,3H); 4,97-5,07 (m,2H); 5,45 (m,2H); 5,70-5,90 (m,1H). Massenspektrum: 154 ($M^+$, 1), 122 (6), 95 (23), 94 (36), 81 (36), 80 (89), 79 (100), 74 (38), 71 (14), 67 (34), 59 (24), 53 (30), 43 (46), 41 (64), 39 (49).

In den nachfolgenden Beispielen 2-8 wurde der jeweilige Akkord in üblicher Weise mittels Riechstreifen abgerochen und so verglichen. Zusätzlich wurde im Aromabeispiel 5 und dem entsprechenden Vergleichsbeispiel 8 das Nahrungsmittel verkostet. Es wurden dabei die gleichen Aspekte wie auf den Riechstreifen festgestellt.

II. Formulierungsbeispiele

Beispiel 2

Parfümerie-Akkord orientalischer Richtung

| | Akkord | |
|---|---|---|
| | (a) | (b) |
| | (Gewichtsteile) | |
| Benzylacetat | 30 | 30 |
| Linalylacetat | 20 | 20 |
| p-tert.Butylcyclohexylacetat | 80 | 80 |
| Phenylethylacetat | 45 | 45 |
| α-Hexylzimtaldehyd | 80 | 80 |
| Decanal 10% in DPG | 3 | 3 |
| 10-Undecenal 10% in DPG | 4 | 4 |
| 2-Methylundecanal 10% in DPG | 2 | 2 |
| Cyclamenaldehyd* | 5 | 5 |
| Bergamottöl, rekonstruiert | 85 | 85 |
| Sandelholzöl | 25 | 25 |
| Zibet-Tinktur, rekonstruiert 10% in DPG | 2 | 2 |
| Coumarin* | 25 | 25 |
| Cyclohexal* | 15 | 15 |
| Dihydromyrcenol* | 10 | 10 |
| Dodecenal 1% in DPG | 5 | 5 |
| Estragol 10% in DPG | 1 | 1 |
| Eugenol | 1,5 | 1,5 |
| Evernyl* | 1 | 1 |
| Galaxolid* 50% in DEP | 60 | 60 |
| Gardenol* | 8 | 8 |
| Geraniol | 30 | 30 |
| Hedion* | 5 | 5 |
| Indolen* | 1 | 1 |
| β-Jonon | 10 | 10 |
| Iso-E-super* | 5 | 5 |
| Isoraldein* 70 | 30 | 30 |
| Jasmon* | 1 | 1 |
| Lilial* | 40 | 40 |
| Linalool | 60 | 60 |
| Methyldiantilis* | 0,5 | 0,5 |
| Methyllaiton* 10% in DPG | 2 | 2 |
| Orangenöl-Terpene, destilliert | 80 | 80 |
| γ-Undecalacton | 3 | 3 |
| γ-Nonalacton | 5 | 5 |
| Rosenoxid* 10% in DPG | 3 | 3 |

(fortgesetzt)

| | Akkord | |
|---|---|---|
| | (a) | (b) |
| | (Gewichtsteile) | |
| Benzylsalicylat | 50 | 50 |
| Sandalore* | 35 | 35 |
| Stemon* | 5 | 5 |
| α-Terpineol | 15 | 15 |
| Vanillin | 10 | 10 |
| Vertofix* coeur | 50 | 50 |
| Dipropylenglykol | 52 | 44 |
| Verbindung **1** | --- | 10 |
| | 1000 | 1000 |

Der Parfümerie-Akkord (a) ist durch einen orientalischen Charakter geprägt. Der Zusatz von 10 Teilen der Verbindung **1** im Parfümerie-Akkord (b) verleiht diesem eine angenehme, natürlich wirkende Frische und überraschenderweise wird gleichzeitig der blumige Aspekt vorteilhaft unterstrichen.

Beispiel 3

Parfümerie-Akkord frisch-blumiger femininer Richtung

| | Akkord | |
|---|---|---|
| | (a) | (b) |
| | (Gewichtsteile) | |
| Benzylacetat | 5 | 5 |
| Linalylacetat | 80 | 80 |
| Phenylethylalkohol | 20 | 20 |
| Methylanthranilat 10% in DPG | 5 | 5 |
| Bergamottöl | 100 | 100 |
| Calone 1951* 10% in DPG | 5 | 5 |
| l-Carvon 10% in DPG | 2 | 2 |
| Cetalox* | 5 | 5 |
| Coumarin* | 3 | 3 |
| Cyclogalbanat* | 3 | 3 |
| α-Damascon | 2 | 2 |
| Dihydromyrcenol* | 50 | 50 |
| Ebanol* | 5 | 5 |
| Evernyl* | 5 | 5 |
| Floralozon* 10% in DPG | 5 | 5 |
| Galaxolid* 50% in DEP | 110 | 110 |
| Geraniumöl | 2 | 2 |
| Hedion* | 200 | 200 |
| cis-3-Hexenol | 1 | 1 |
| Indol* 1% in DPG | 2 | 2 |
| β-Jonon | 25 | 25 |
| Iso-E-super* | 45 | 45 |
| Jasmon*10% in DPG | 8 | 8 |
| Lilial* | 20 | 20 |
| Linalool | 80 | 80 |

(fortgesetzt)

| | Akkord | |
|---|---|---|
| | (a) | (b) |
| | (Gewichtsteile) | |
| Orangenöl | 40 | 40 |
| Rosoflor 2* | 5 | 5 |
| cis-3-Hexenylsalicylat | 5 | 5 |
| Scentenal* 1% in DPG | 2 | 2 |
| Tropional* | 30 | 30 |
| Vertofix* coeur | 10 | 10 |
| Dipropylenglykol | 120 | 115 |
| Verbindung **1** | --- | 5 |
| | 1000 | 1000 |

Der obige Parfümerie-Akkord (a) ist durch einen frisch-blumigen Charakter geprägt und wirkt gesamthaft betrachtet feminin. Der Zusatz von 5 Teilen der Verbindung **1** in Parfümerie-Akkord (b) belebt diesen durch eine angenehme Frische und lässt ihn voller und abgerundeter hervortreten. Zudem macht sich ein angenehmer grün-fruchtiger Aspekt bemerkbar.

Beispiel 4

Parfümerie-Akkord frisch-blumiger Richtung mit grün-fruchtigen Aspekten

| | Akkord | |
|---|---|---|
| | (a) | (b) |
| | (Gewichtsteile) | |
| Benzylacetat | 60 | 60 |
| Dimethylbenzylcarbinylacetat | 25 | 25 |
| Geranylacetat | 40 | 40 |
| Phenylethylalkohol | 100 | 100 |
| α-Hexylzimtaldehyd | 100 | 100 |
| 10-Undecenal | 0,3 | 0,3 |
| Phenylacetaldehyd | 2 | 2 |
| Bergamottöl, rekonstruiert | 100 | 100 |
| Cyclohexal* | 40 | 40 |
| Fixolid* | 70 | 70 |
| Geraniol | 50 | 50 |
| Nelkenöl | 7 | 7 |
| Hedion* | 50 | 50 |
| Heliotropin | 10 | 10 |
| Isoeugenol | 2 | 2 |
| Isoraldein* 95 | 40 | 40 |
| Lilial* | 40 | 40 |
| Linalool | 50 | 50 |
| Mandarinenöl | 20 | 20 |
| γ-Undecalacton | 1 | 1 |
| Benzylsalicylat | 40 | 40 |
| cis-3-Hexenylsalicylat | 10 | 10 |
| Tropional* | 10 | 10 |
| Dipropylenglykol | 132,7 | 129,7 |

(fortgesetzt)

| | Akkord | |
|---|---|---|
| | (a) | (b) |
| | (Gewichtsteile) | |
| Verbindung **1** | ---<br>1000 | 3<br>1000 |

Der Parfümerie-Akkord (a) ist durch eine frisch-blumige Note dominiert, zeigt aber auch grün-fruchtige Aspekte. Der Zusatz von 3 Teilen der Verbindung **1** im Parfümerie-Akkord (b) bereichert diesen durch eine angenehme, natürlich wirkende Frische, macht ihn gleichzeitig voller und eleganter und betont eine auffallende, Interesse erwekkende, an Birnen erinnernde Note.

Beispiel 5

Aroma vom Typ Birne

| | Akkord | |
|---|---|---|
| | (a) | (b) |
| | (Gewichtsteile) | |
| Isoamylacetat | 20 | 20 |
| Hexylacetat | 40 | 40 |
| Geranylacetat | 2 | 2 |
| Heptylacetat | 5 | 5 |
| Ethylbutyrat | 10 | 10 |
| trans-2-Hexenal | 5 | 5 |
| Amylbutyrat | 10 | 10 |
| Hexanol | 5 | 5 |
| Vanillin | 3 | 3 |
| Verbindung **1** | --- | 4 |
| Propylenglykol | 900 | 896 |
| | 1000 | 1000 |

Der Zusatz von 4 Teilen der Verbindung **1** zum Aroma (a) wirkt sich sehr vorteilhaft aus, indem der Eindruck einer vollreifen, trotzdem aber erfrischenden Birne erweckt wird.

Ein derartiges Aroma, d.h. Aroma (b), wurde in einem Nahrungsmittelfertigprodukt, nämlich Joghurt, zugesetzt, wobei die Verbindung **1** in einer Konzentration von 0,1 bis 100 ppm (als konzentrierte Base) vorlag. Insbesondere bei einer Konzentration von 0,1 bis 10 ppm kam der vollreife erfrischende Birnenaspekt voll zur Geltung.

III. Vergleichsbeispiele

Beispiel 6

Wenn in Beispiel 2 die Verbindung 1 durch dieselbe Menge der Verbindung 4 ersetzt wird, kommt die attraktive Wechselwirkung zwischen p-tert.-Butylcyclohexylacetat, Phenylethylacetat, Stemon und der erfindungsgemässen Verbindung 1, die in der Komposition durch eine sehr angenehme, natürlich wirkende Frische zum Ausdruck kommt, nicht zustande. Die Komposition wirkt in der Kopfnote viel weniger entwickelt.

Beispiel 7

Wenn in Beispiel 3 die Verbindung 1 durch dieselbe Menge der Verbindung 4 ersetzt wird, fehlt diesem so modifizierten Parfümerie-Akkord die mit der Verbindung 1 erzielte angenehme Frische und der mit der Verbindung 1 erhaltene wünschenswerte grün-fruchtige Aspekt wird durch einen wesentlich weniger attraktiven allgemein-fruchtigen

Aspekt ersetzt.

Beispiel 8

Wenn in Beispiel 5 Verbindung 1 durch Verbindung 4 ersetzt wird, fehlt dem so erhaltenen Birnenaroma der Frische-Aspekt.

Zusammenfassend ergibt sich aus den Vergleichsbeispielen, dass Verbindung 1 generell nicht durch Verbindung 4 ersetzt werden kann, denn bei Ersatz der Verbindung 1 durch die Verbindung 4 fehlt der wünschenswerte Frische-Aspekt, der oft von grün-fruchtigen Noten begleitet wird, zur Gänze.

## Patentansprüche

1. **1.** Methyl (E)-4,7-octadienoat

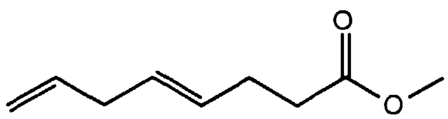

**1**

2. **2.** Verfahren zur Herstellung von Methyl (E)-4,7-octadienoat (**1**), gekennzeichnet durch Orthoester-Claisen-Umlagerung von 1,5-Hexadien-3-ol (**2**) mit Trimethylorthoacetat (**3**) in Anwesenheit einer Carbonsäure C1 bis C8 gemäss der Gleichung

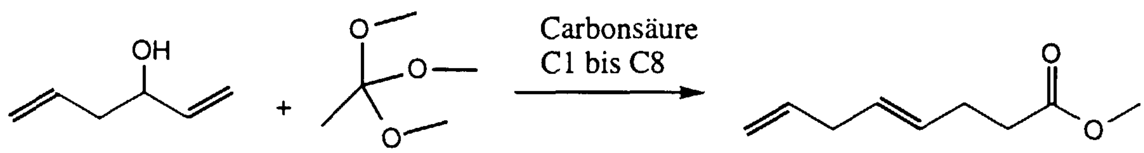


**2** **3** **1**

3. **3.** Verfahren nach Anspruch 2, dadurch gekennzeichnet, dass die Carbonsäure Propionsäure ist.

4. **4.** Verwendung von Methyl (E)-4,7-octadienoat als Riech- und/oder Geschmackstoff (**1**).

5. **5.** Riech- und/oder Geschmackstoffkomposition enthaltend Methyl (E)-4,7-octadienoat.

6. **6.** Riech- und/oder Geschmackstoffkomposition enthaltend 0,1 bis etwa 60 Gew.%o Methyl (E)-4,7-octadienoat (**1**).

7. **7.** Riech- und/oder Geschmackstoff enthaltend 3 bis 10 Gew.%o Methyl (E)-4,7-octadienoat (**1**).

8. **8.** Nahrungsmittelfertigprodukt enthaltend 0,01 bis 100 ppm Methyl (E)-4,7-octadienoat (**1**).

9. **9.** Nahrungsmittelfertigprodukt enthaltend 0,1 bis 10 ppm Methyl (E)-4,7-octadienoat (**1**).

## Claims

1. **1.** Methyl (E)-4,7-octadienoate

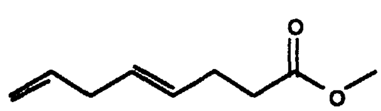

1

2. A process for the manufacture of methyl (E)-4,7-octadienoate (1), characterized by the Claisen orthoester rearrangement of 1,5-hexadien-3-ol (2) with trimethyl orthoacetate (3) in the presence of a Cl to C8 carboxylic acid in accordance with the equation

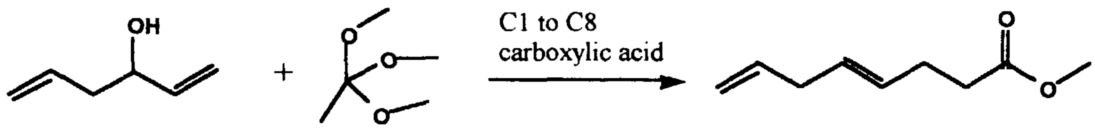


2 3 1

3. A process according to claim 2, characterized in that the carboxylic acid is propionic acid.

4. The use of methyl (E)-4,7-octadienoate as an odorant and/or flavorant (**1**).

5. An odorant and/or flavorant composition containing methyl (E)-4,7-octadienoate.

6. An odorant and/or flavorant composition containing 0.1 to about 60 wt.‰ methyl (E)-4,7-octadienoate (**1**).

7. An odorant and/or flavorant containing 3 to 10 wt.‰ methyl (E)-4,7-octadienoate (1).

8. A finished nutrient product containing 0.01 to 100 ppm methyl (E)-4,7-octadienoate (1).

9. A finished nutrient product containing 0.1 to 10 ppm methyl (E)-4,7-octadienoate (1).

**Revendications**

1. (E)-4,7-octadiénoate de méthyle

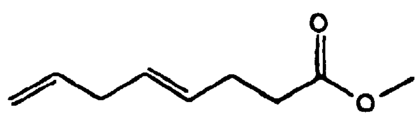

1

2. Procédé de préparation de (E)-4,7-octadiénoate de méthyle (1), caractérisé en ce qu'on effectue une transposition d'orthoester de Claisen de 1,5-hexandiène-3-ol (2) avec de l'orthoacétate de triméthyle(3) en présence d'un acide carboxylique en $C_1$ à $C_8$ selon l'équation

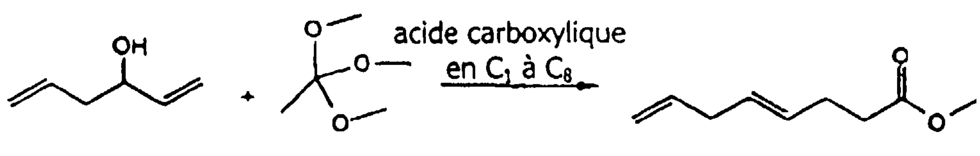


2 3 1

3. Procédé selon la revendication 2, caractérisé en ce que l'acide carboxylique est l'acide propionique.

4. Utilisation du (E)-4,7-octadiénoate de méthyle comme parfum et/ou arôme.

5. Composition de parfum et/ou d'arôme contenant le (E)-4,7-octadiénoate de méthyle.

6. Composition de parfum et/ou d'arôme contenant de 0,1 à environ 60‰ en poids de (E)-4,7-octadiénoate de méthyle

(1).

7. Composition de parfum et/ou d'arôme contenant de 3 à 10‰ en poids de (E)-4,7-octadiénoate de méthyle (1).

8. Produit fini alimentaire contenant de 0,01 à 100 ppm de (E)-4,7-octadiénoate de méthyle (1).

9. Produit fini alimentaire contenant de 0,1 à 10 ppm de (E)-4,7-octadiénoate de méthyle (1).